# EUROPEAN PATENT APPLICATION

(11) **EP 4 317 401 A1**
(43) Date of publication of application: **07.02.2024**
(21) Application number: 22188233.5
(22) Date of filing: 02.08.2022
(51) Int. Cl.: C12M 1/12, C12N 11/08

(54) **DEVICE CONTAINING A MACROPOROUS ELASTOMERIC MATERIAL FOR THE ENRICHMENT AND CULTIVATION OF MICROORGANISMS IN SITU**

(71) Applicant: Karlsruher Institut für Technologie, 76131 Karlsruhe (DE)
(72) Inventor: NIEMEYER, Christof, 28213 Bremen (DE); RABE, Kersten, 76137 Karlsruhe (DE); ZOHEIR, Ahmed, Beni Suef (EG)

(57) **Abstract**

The present invention relates to a device (1) containing a macroporous elastomeric material (2) for the enrichment and cultivation of microorganisms *in situ* and to the application of said device for sampling, colonization and isolation of even previously uncultivable or unknown microorganisms in a wide variety of habitats.

## Description

The present invention relates to a device containing a macroporous elastomeric material for the enrichment and cultivation of microorganisms *in situ* and to the application of said device for sampling, colonization and isolation of even previously uncultivable or unknown microorganisms in a wide variety of habitats.

Natural evolution has produced an almost infinite variety of microorganisms that can colonize almost any conceivable habitat in complex communities.

The vast majority of these microbial consortia are, however, uncharacterized and cannot be cultured in the laboratory. Modern methods of high-throughput nucleic acid sequencing are beginning to allow us to better understand the enormous complexity of this so-called "microbial dark matter" (C. Rinke et al., Nature 2013, 499 (7459), 431-437), however, although a surprisingly large number of branches are already visible, the full extent of the microbial tree of life is still difficult to imagine (L. A. Hug et *al.,* Nat Microbiol 2016, 1 (5), 16048). This lack of knowledge is very unfortunate, as it is becoming increasingly clear that microbial communities not only play an important role in human health and our environment (L. Hall-Stoodley et al., Nat Rev Microbiol 2004, 2 (2), 95-108), but could also serve as invaluable tools for the next generation of biotechnological processes (W. Verstraete, Microb Biotechnol 2015, 8 (1), 36-37). Therefore, there is a growing interest in efficient strategies to isolate novel microorganisms from complex habitats in order to exploit the biological materials and information they contain, for example specific proteins or metabolic pathways, for biotechnological applications (W. H. Lewis et al., Nat Rev Microbiol 2021, 19 (4), 225-240). To improve the accessibility of non-cultivable microorganisms for technical cultivation, the so-called "isolation chip" (ichip) platform had been developed to isolate previously non-cultivable microorganisms from environmental samples by allowing exposure to natural growth factors through *in situ* culture (B. Berdy et al., Nat Protocols 2017, 12 (10), 2232-2242). To achieve this, cultivation was moved to the microbes' natural habitat by placing cells taken from various environmental samples into miniaturized diffusion chambers of a chip comprising porous polycarbonate membranes, which was then returned to nature for incubation. Although the ichip platform increases microbial biomass from, for example, soil samples by 5- to 300-fold, depending on the study, the technology is cumbersome because environmental samples must first be collected, then processed for integration into the chip, and the chips thus processed must be re-incubated in the original habitat. Hence, with the ichip platform it is not feasible to sample, for example, aqueous and/or dynamic habitats, such as moving bed biofilters or wastewater treatment plants.

Although a variety of hydrophobic micro-, meso- or macroporous materials suitable for the isolation of microorganisms, like zeolites, active carbon, porous silicates, hybrid metal-organic frameworks and hypercrosslinked porous polymers, exist, several macroporous elastomeric materials combine the features of being, e.g., easy to process, being moldable and gas permeable. Polydimethylsiloxane (PDMS), as an example for a low-cost macroporous elastomeric material, has the further advantages of being biochemically inert and diversely modifiable in terms of its surface properties through well-controlled silane chemistry. The production of foam- and sponge-like porous silicone has been well studied, and it has already been shown that these materials can be used for applications as oil absorbers, heterogeneous catalysts, actuators and sensors, or even for tissue engineering of animal cells (D. Zhu et al., J. Mater. Chem. A 2017, 5 (32), 16467-16497) .

However, the suitability of macroporous elastomeric materials for the targeted enrichment and cultivation of microorganisms *in situ* has neither been discussed nor shown before.

Thus, the objective technical problem was to provide a device containing a macroporous elastomeric material that is suitable for sampling, enrichment and cultivation of microorganisms *in situ* in a wide variety of habitats, like aqueous or dry and/or dynamic habitats.

The objective technical problem is solved by the embodiments characterized in the independent claims. Preferred embodiments can be found in the dependent claims.

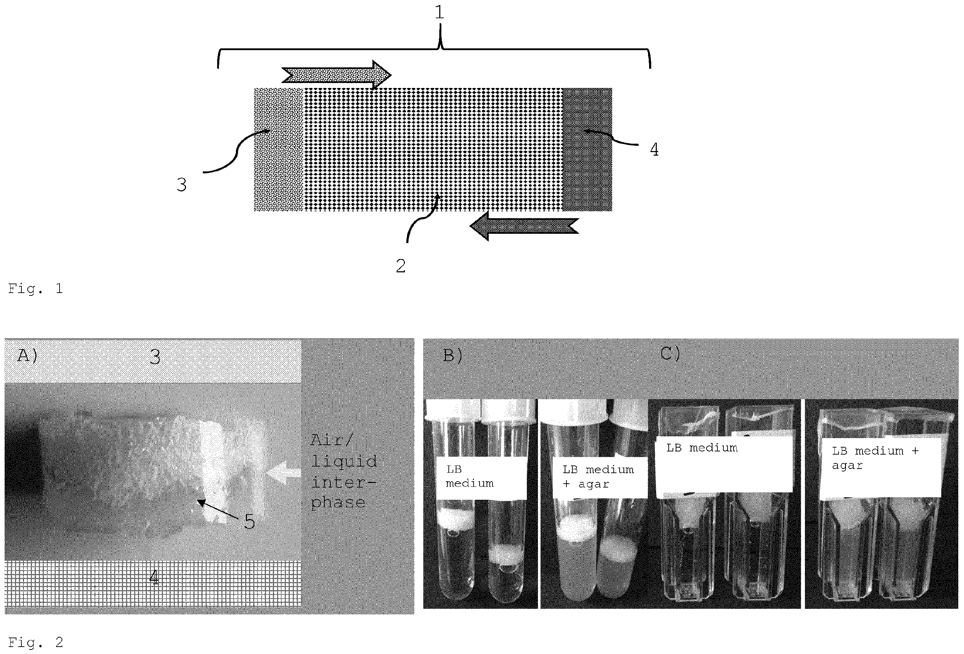

The invention describes a device (1) containing a macroporous elastomeric material (2) that can be used for the enrichment, colonization and isolation of also previously uncultivable or unknown microorganisms *in situ* from arbitrary habitats (figure 1) .

According to the invention, *in situ* refers to the isolation, enrichment, colonization and cultivation of microorganisms in the original habitat without processing the sample of microorganisms after collection *in vitro* and reincubation in the original habitat.

The macroporous elastomeric material (2) according to the invention is any material exhibiting elastic or rubber-like properties and having an adjustable porosity, containing physically interconnected pores of an average pore size ranging from 0.1 - 950 µm, preferably from 10 - 300 µm.

The term "interconnected pores" refers to an open porosity, wherein a fluid flow is effectively taking place as distinguished from closed pores as non-connected cavities (https:// en.wikipedia.org/wiki/Porosity). Thus, the interconnected pores according to the invention form a continuous network of connected channels throughout the macroporous elastomeric material (2).

The macroporous elastomeric material (2) within the device (1) is additionally characterized by flexibility, moldability, gas permeability and adjustable surface properties.

In a first aspect of the invention is provided a device (1) comprising a macroporous elastomeric material (2) as a macroporous matrix connected via open, interconnected pores to a first donor site (3) and a second donor site (4) (figure 1), wherein the first donor site (3) provides contact to an aqueous, solid or gaseous phase containing microorganisms, and the second donor site (4) provides contact to an aqueous, solid or gaseous phase, optionally containing nutrients, a culture medium, attractants, repellents and/or other molecular as well as cellular interaction partners for the microorganisms to be collected and enriched from the first donor site (3). For example, microalgae or cyanobacteria cultured in the second donor site (4) and metabolites secreted by the microalgae or cyanobacteria can serve as cellular or molecular interaction partners, respectively.

According to the invention, the term "first donor site" is to be regarded as an entry point for microorganisms, such as bacteria, archaea or microbial eukaryotes (e.g., microalgae), and the term "second donor site" is to be regarded as a nutrient reservoir or culture medium for the macroporous elastomeric material (2) and the microorganisms to be cultured within the macroporous elastomeric material (2).

The first donor site (3) is, e.g., a surface suitable for contact with the environment, such as a cover lid provided with environmental contact holes, the second donor site (4) is, e.g., a reservoir or a bioreactor.

The first donor site (3) provides contact to any liquid, solid or gaseous environment containing microbial cells, e.g., airborne habitats, soils or biofilters. In the device (1) cells included in gaseous, liquid, solid or other habitat environments can enter the macroporous elastomeric material (2) from the first donor site (3) and move by diffusion, motility or propagation inside and towards the direction of the second donor site (4). Substances contained in the medium of the second donor site (4) can diffuse within the macroporous elastomeric material (2) towards the first donor site (3) via active or passive diffusion, thereby creating gradients of substances with high and low substance concentrations near the second donor site (4) and the first donor site (3), respectively.

The substance gradients affect the growth, accumulation and establishment of cultures of microbial cells that migrate from the first donor site (3) to the second donor site (4), leading to the formation of stable microbial communities in the macroporous elastomeric material (2).

By modifying the substances added to the second donor site (4) or by creating a specific gas atmosphere in the second donor site (4), like a hydrogen-, methane- or carbon dioxide-enriched gaseous phase, enrichment, colonization and isolation of selected microorganisms, which are attracted by the substances in the second donor site (4), is possible.

In a preferred embodiment the macroporous elastomeric material (2) contained in the device (1) is a macroporous elastomeric silicone foam (MESIF) (5).

A foam belongs to the group of elastomeric materials and is a lightweight, open-cell product of low density to allow air movement through the cell structure (D. Weaire et al., "The Physics of Foams", Oxford University Press, 1999, ISBN 0198510977, ISBN 978-0-1985-1097-0; I. Cantat et al., "Foams: structure and dynamics", Oxford University Press, ed. S.J. Cox, 2013, ISBN 9780199662890).

A silicone is defined as a polymer made up of siloxane (-R₂Si-O-SiR₂-, where R is an organic group) .

The macroporous elastomeric silicone foam (4) has, according to the above specifications, an adjustable porosity, containing physically interconnected pores of an average pore size ranging from 0.1 - 950 µm, preferably from 10 - 300 µm.

In a preferred embodiment the macroporous elastomeric silicone foam (5) contains Polydimethylsiloxane (PDMS).

In a particular embodiment the macroporous elastomeric silicone foam (5) contains other materials beside Polydimethylsiloxane (PDMS) selected from other hydrophobic or hydrophilic polymers or blockcopolymers, such as polypropylene, polyethylene, polyamides, polyesters or blockcopolymers thereof, and/or particles made of polymers, metals, oxides or minerals are additionally added.

In a particular embodiment the surface of the macroporous elastomeric silicone foam (5) is chemically modified with established organosilane chemistry.

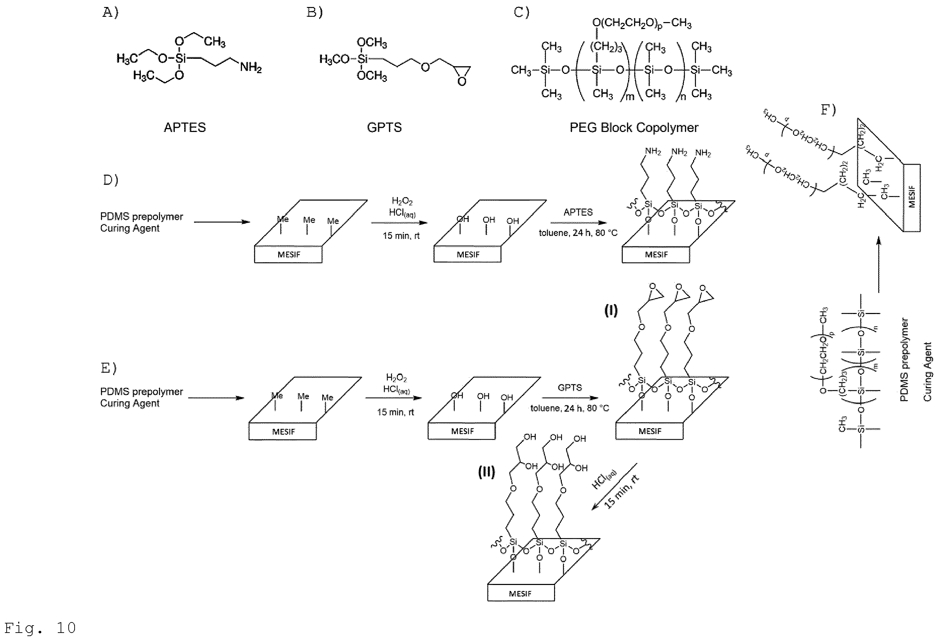

In a preferred embodiment the surface of the macroporous elastomeric silicone foam is modified with primary amino groups, epoxy groups or hydrophilic polyethylenglycol (PEG) moieties (figure 10).

Surface modification with functional groups, such as amino-, hydroxyl- or PEG groups significantly affect the passive diffusional transport of small molecules through the macroporous elastomeric silicone foam (5). Such surface modifications can also be used to influence the colonization of macroporous elastomeric silicone foam surfaces by different bacterial communities, as it is well known that the colonization of surfaces can be strongly influenced by their polarity, hydrophobicity, and micro- and nanotopography (R. M. Donlan et al., Emerg Infect Dis 2002, 8 (9), 881-890; H. C. Flemming et al., Nat Rev Microbiol 2016, 14 (9), 563-575).

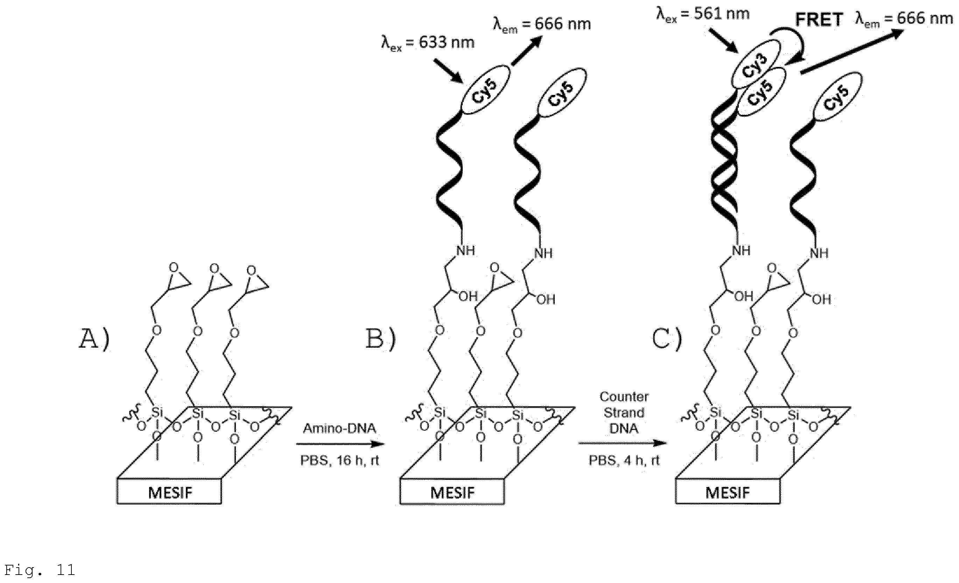

In another embodiment even large functional macromolecules, such as DNA oligonucleotides, are installed on the surface of the macroporous elastomeric silicone foam (4) (figure 11).

In another embodiment, following the procedure described in figure 11 for installing DNA oligonucleotides, proteins, such as enzymes, antibodies or other binding proteins, are installed on the surface of the macroporous elastomeric silicone foam (5) to affect the colonization, growth and maintenance of microbial communities.

In one embodiment the device (1) containing a macroporous elastomeric material (2) is a chip for the enrichment and cultivation of microorganisms *in situ,* applicable in various exemplary habitats. In this embodiment a surface suitable for contact with the environment represents the first donor site (3) and a reservoir enclosed by the chip represents the second donor site (4), filled, e.g., with a culture medium supplemented with attractants or repellents.

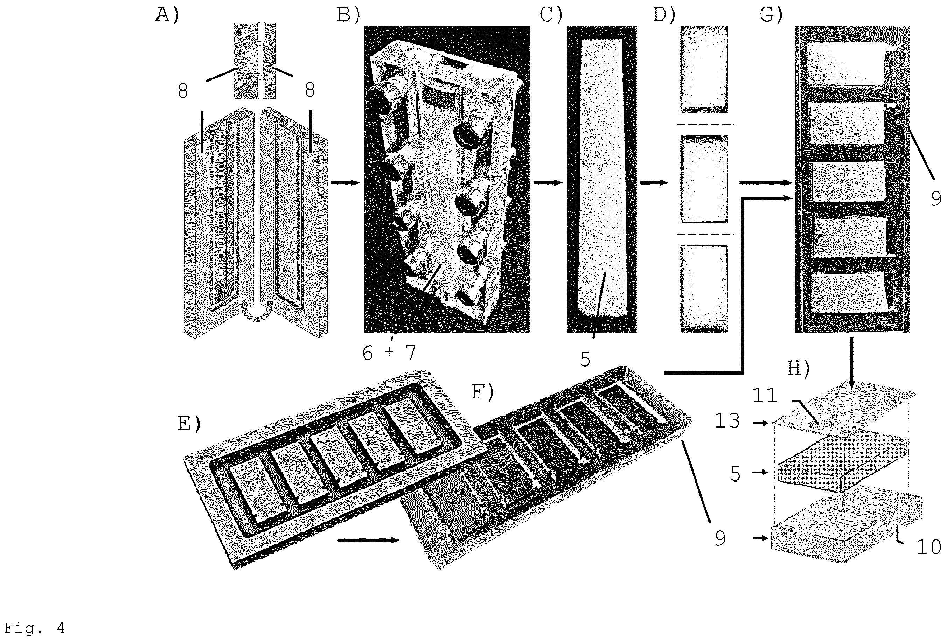

In a preferred embodiment the chip according to the invention is a chip (12) containing at least one piece of a macroporous elastomeric silicone foam (5) (MESIF-chip) and comprises a PDMS housing structure (9), at least one piece of a macroporous elastomeric silicone foam (5) and a PDMS cover lid (13) into which environmental contact holes (11) are punched (figure 4). The PDMS housing structure (9) according to the invention includes a reservoir (10) for fitting at least one piece of the macroporous elastomeric silicone foam (5), into which nutrient medium or substrates can be placed. In this embodiment the PDMS cover lid (13) into which environmental contact holes (11) are punched represents the first donor site (3) and the reservoir (10) represents the second donor site (4).

In another embodiment the device (1) containing a macroporous elastomeric material (2) is a functional interface, like a membrane, in bioreactors, tubular reactors or flat-bed reactor. In a specific embodiment the device (1) containing a macroporous elastomeric material (2) as functional interface is integrated in a bioreactor for cultivating photosynthesis-active microorganisms, such as algae or cyanobacteria.

In this embodiment the membrane surface itself represents the first donor site (3) and the respective reactor represents the second donor site (4).

For sampling microorganisms *in situ* in an, e.g., marine environment, the device (1) according to the invention is a functional interface of a bioreactor. In this example microorganisms can migrate/diffuse from the surrounding marine habitat via the first donor site (3) into the macroporous elastomeric material (2) to form a synergistic community with the aquatic organisms such as microalgae, fish or crustaceans, contained together with an aqueous nutrient medium in the second donor site (4). From the second donor site (4), a gradient of nutrient components from the aquatic organism culture is created by diffusion into the macroporous elastomeric material (2).

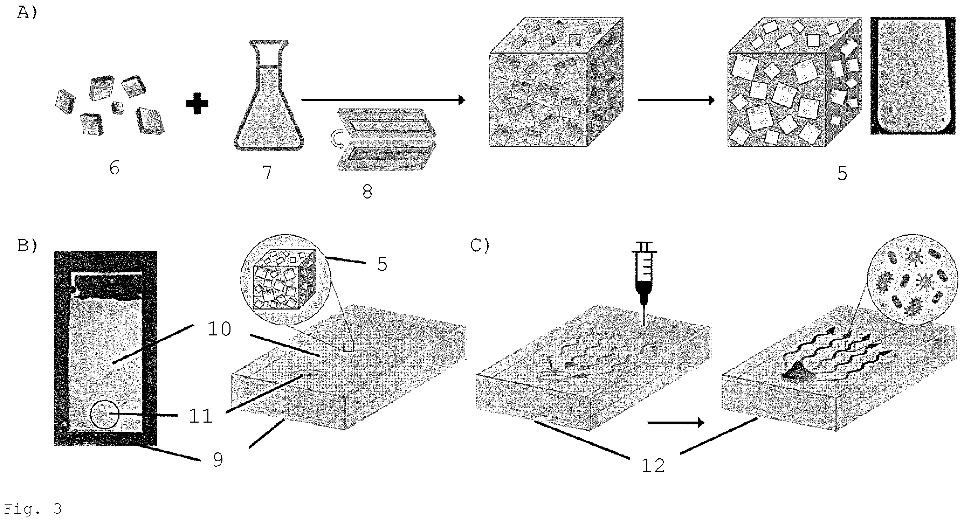

In another aspect of the invention, the manufacturing process for a macroporous elastomeric silicone foam (5) is described. The production of the macroporous elastomeric silicone foam (5) comprises the following steps (figure 3A):
A) Mixing porogen crystals (6) with a grain size of 0.1 µm - 950 µm with a silicone prepolymer solution (7);
B) Pouring the resulting suspension of step A) into a casting mold (8);
C) Centrifugation of the filled mold of step B) to disperse and pack the porogen crystals (6) within the liquid silicone prepolymer;
D) Thermal curing of the suspension of step C);
E) Releasing the cured macroporous elastomeric silicone foam from the casting mold (8);
F) Leaching the macroporous elastomeric silicone foam from step E) to dissolve the integrated porogen crystals (6);
G) Drying the molded macroporous elastomeric silicone foam;
H) Optionally modifying the surface of the macroporous elastomeric silicone foam (5) after step G) by means of organosilane chemistry.

The porogen crystals (6) in step A) are selected from water dissolvable molecules, such as crystals of sodium chloride, calcium chloride, magnesium chloride or other inorganic salts, or crystals of sucrose, glucose, fructose or amino acids.

In a preferred embodiment sodium chloride crystals or sucrose crystals are used as porogen crystals (6).

The grain size of the porogen crystals (6) ranges from 0.1 µm - 950 µm, preferably from 10 - 300 µm.

The grain size is defined as the diameter of singular sediment grains. This differs from the size of a crystallite, which refers to the size of a single crystal inside a particle or grain.

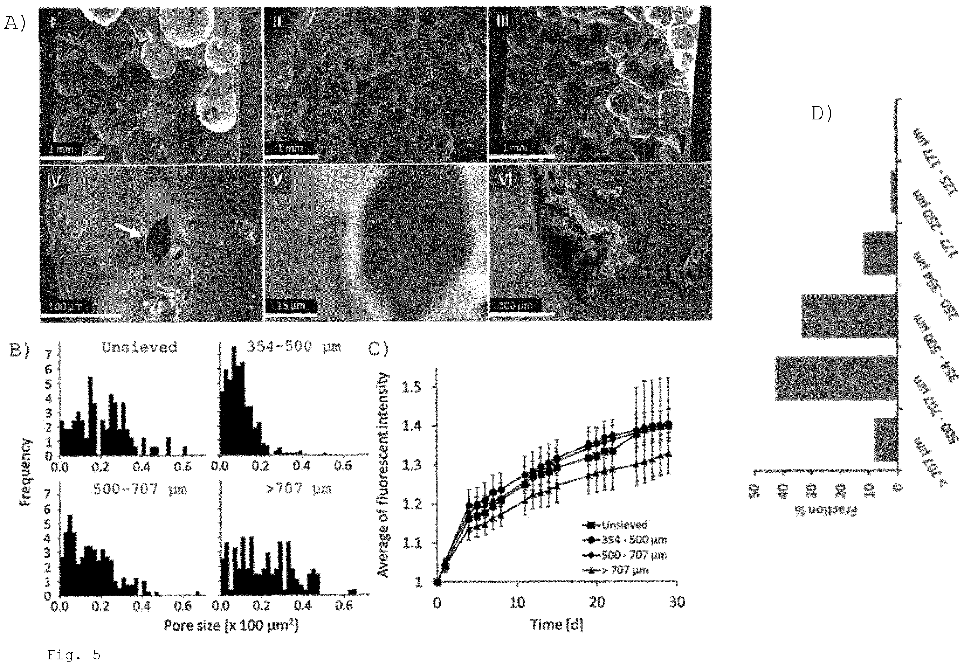

The porosity of the macroporous elastomeric silicone foam (5) to be produced is adjusted by using different grain size fractions of porogen crystals (6) (figure 5B)); example 2).

In a preferred embodiment the silicone prepolymer solution (7) in step A) is a mixture of silicone oligomers with a curing agent in a ratio of 10:1 (w/w) . In a more preferred embodiment the curing agent is Sylgard 184 (Down Corning).

In a preferred embodiment the silicone in the silicone prepolymer solution (7) is Polydimethylsiloxane (PDMS).

The casting mold (8) in step B) is preferably made of polymethyl methacrylate (PMMA). The casting mold (8) preferable consists of two pieces of rectangular geometry, with edges, grooves and a sealing film, like a PTFE (Polytetrafluorethylen) film or Parafilm^{®} M, in between, is closed by screws, that is made for centrifugal mixing of the porogen crystals (6) and the liquid silicone (figures 4A), 4B)).

Centrifugation in step C) is preferably done for 20 minutes, at 5 °C and 4600 rpm.

Thermal curing in step D) is preferable performed at 70 °C for 1 hour.

In step F), the resulting molded macroporous elastomeric silicone foam is preferably mechanically compressed several times in a water bath to dissolve the integrated porogen crystals, and thereby a macroporous structure of interconnected pores within the elastomer is created.

Another way of leaching according to the invention is soaking of the resulting molded macroporous elastomeric silicone foam for several hours in a 40°C water bath with or without sonication. In step G), drying is preferably performed at 90 °C for one hour. After step G), in a preferred embodiment the surface of the prepared silicone foam is modified by means of established organosilane chemistry (example 3). In a more preferred embodiment the surface of the macroporous elastomeric silicone foam (5) is modified with primary amino groups or expoxy groups using APTES ((3-Aminopropyl) triethoxysilane) or GPTS (3-Glycidyloxypropyl) trimethoxysilane), respectively (figure 10). For a surface modification with hydrophilic polyethylenglycol (PEG) moieties, in step A) the silicone prepolymer solution (7) is a mixture of silicone oligomers and a thermal curing agent in a ratio of 10:1 (w/w), where a silicone-PEG block copolymer, like a PDMS-PEG block copolymer, is added in a ratio of 0.25 % (w/w), related to the total mass of the silicone prepolymer solution (7) (example 3).

In another preferred embodiment the surface of the macroporous elastomeric silicone foam (5) is modified with even large functional macromolecules, such as DNA oligonucleotides (figure 11, example 3) or proteins, such as enzymes, antibodies or other binding proteins.

In a further aspect of the invention, the manufacturing process for a chip (12) containing at least one piece of a macroporous elastomeric silicone foam (5) is described.

The manufacturing process of a chip (12) containing at least one piece of a macroporous elastomeric silicone foam (5) comprises the following steps:
I) Fabricating a PDMS housing structure (9) with a reservoir (10) suitable for holding at least one piece of a macroporous elastomeric silicone foam (5), using a suitable casting mold;
J) Fabricating a PDMS cover lid (13) cast from PDMS, into which environmental contact holes (11) are punched;
K) Cutting a molded macroporous elastomeric silicone foam (5) into pieces to fit into the PDMS housing structure (9) of step I) and inserting at least one piece of a macroporous elastomeric silicone foam (5) into the reservoir (10);
L) Plasma surface activation of the PDMS housing structure (9), containing at least one piece of the macroporous elastomeric silicone foam (5) from step K), and of the PDMS cover lid (13).
M) Covering the housing structure (9) containing at least one piece of the macroporous elastomeric silicone foam (5) with the PDMS cover lid (13);
N) Curing the PDMS housing structure including the cover lid and sealing the PDMS housing structure including the cover lid by bonding.

In a preferred embodiment plasma surface activation in step L) is performed with O₂, N₂ or NH₃ to provide altered surface chemistries that favor or disfavor the enrichment of specific microbial communities. Plasma activation is performed for, e.g., 30 seconds at 300 W and 0.2 mbar.

In a preferred embodiment curing in step N) is performed at 90 °C for 30 minutes.

In a further aspect of the invention the application of the device (1) containing a macroporous elastomeric material (2) is described:
The device (1) containing a macroporous elastomeric material (2) can be used for the enrichment, colonization and isolation of microorganisms *in situ* in a wide variety of habitats. In a specific embodiment, the device (1) containing a macroporous elastomeric material (2) can be used for the enrichment, colonization and isolation of previously uncultivable or unknown microorganisms *in situ* in a wide variety of habitats. The device (1) containing a macroporous elastomeric material (2) can be used in dry, wet and liquid environments, such as indoor air, soils, moving bed biofilters or wastewater treatment plants.

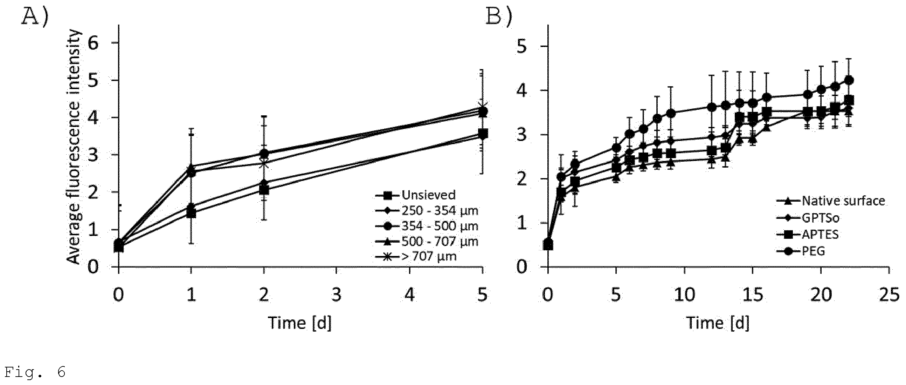

In a preferred embodiment of the invention a chip (12) containing at least one piece of a macroporous elastomeric silicone foam (5) is used for the enrichment, colonization and isolation of microorganisms *in situ* (figures 6-9). In a specific embodiment, the chip (12) containing at least one piece of a macroporous elastomeric silicone foam (5) is used for the enrichment, colonization and isolation of previously uncultivable or unknown microorganisms in a wide variety of habitats. The chip (12) containing at least one piece of a macroporous elastomeric silicone foam (5) can be used in dry, wet and liquid environments, such as indoor air, soils, moving bed biofilters or wastewater treatment plants.

To test the suitability of the chip (12) containing at least one piece of a macroporous elastomeric silicone foam (5) (MESIF-chip) for enriching complex microbial communities from natural habitats first an aerobic moving bed biofilter of a fish farm was chosen. To evaluate the performance of the MESIF material, commercially available polyethylene biofilter carrier chips (Mutag Biochip^{™}) were used as reference material. These chips are reported by the manufacturer to have a high protected active surface area of more than 5,500 m² /m³, and are often used as biocarriers for moving bed biofilm reactor studies (N. H. Dan et al., Sci. Total Environ. 2021, 787, 147680; A. A. Mazioti et al., Water 2021, 13, 1810).

Sterile MESIF-chips (12) and Mutag chips (Mutag Biochip^{™}) were incubated freefloating in the biofilter tank for several days and then collected (example 4B)).

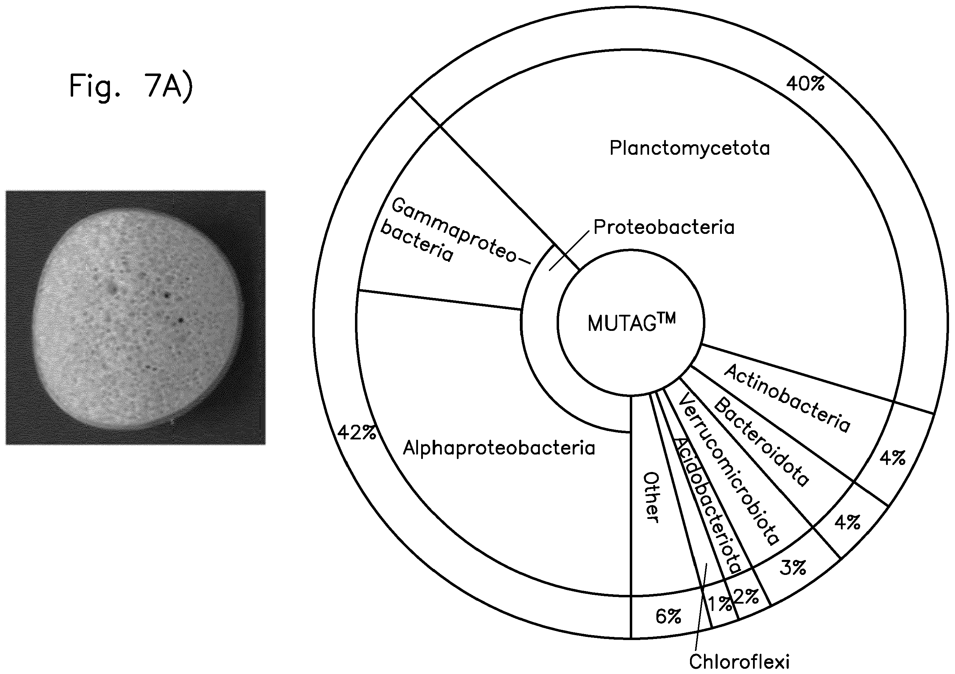

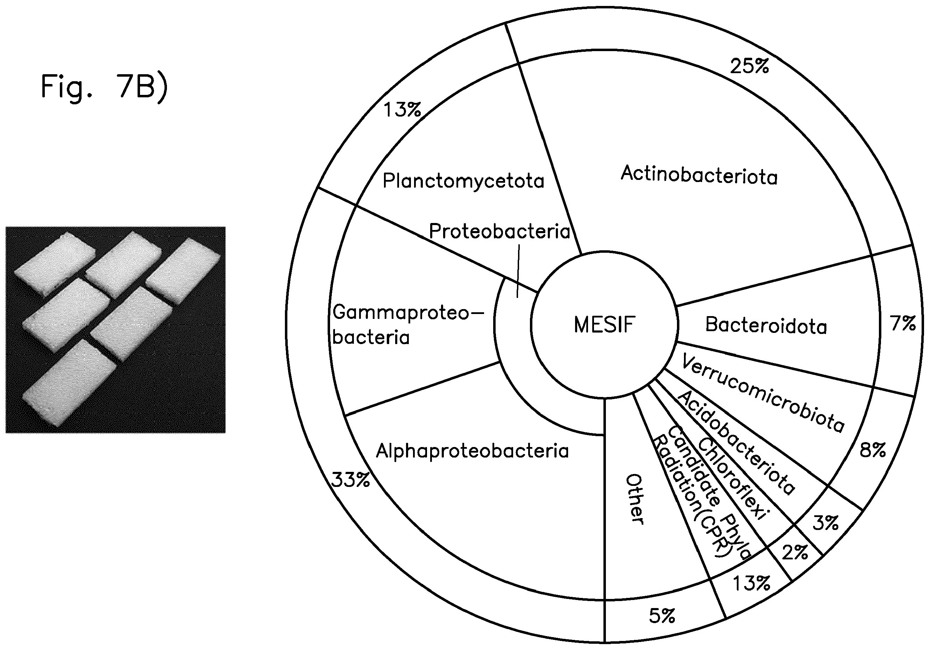

Genomic DNA (gDNA) was extracted from the chips using commercially available extraction kits, and the amount of extracted gDNA was quantified photometrically. After sequencing, the resulting KRONA charts (figures 7A), 7B)) illustrate that the microbial community in the Mutag chips (Mutag Biochip^{™}) was strongly dominated by *Alphaproteobacteria* and *Planctomycetes,* the latter being especially known to form biofilms on various surfaces (S. Wiegand et al., Nat Microbiol 2020, 5 (1), 126-140). In contrast, the MESIF-chip (12) showed a much broader distribution of different bacterial species (figure 7B)). Notably, in the MESIF-chip (12), species from the so called *Candidate Phyla Radiation* (CPR) (S. Wiegand et al., Front. Microbio. 2021, 12, 635506) could be enriched. Based on cultivation-independent approaches, such as 16S rRNA amplicon sequencing, metagenomics and single-cell genomics (A.-K. Kaster et al., Appl. Microbiol. Biotechnol. 2020, 104 (19), 8209-8220) it is currently assumed that this taxon comprises about 70% of the microbial dark matter (L. A. Hug et al., Nat Microbiol 2016, 1 (5), 16048). Many CPR genomes seem to lack ubiquitous biosynthetic pathways, e.g. for the production of amino acids, nucleotides, cofactors and membrane lipids, while protein families associated with cell-cell interactions are frequently found in this group (C. J. Castelle et al., Cell 2018, 172, 1181-1197; R. Méheust et al., Nature communications 2019, 10 (1), 4173). Also, some CPRs seem to live in symbiotic relationships, which could also be the reason why they are so difficult to culture and still very poorly studied.

To test the real-life applicability of a chip (12) containing at least one piece of a macroporous elastomeric silicone foam (5) for environmental issues, secondly sampling of airborne microbial communities was investigated.

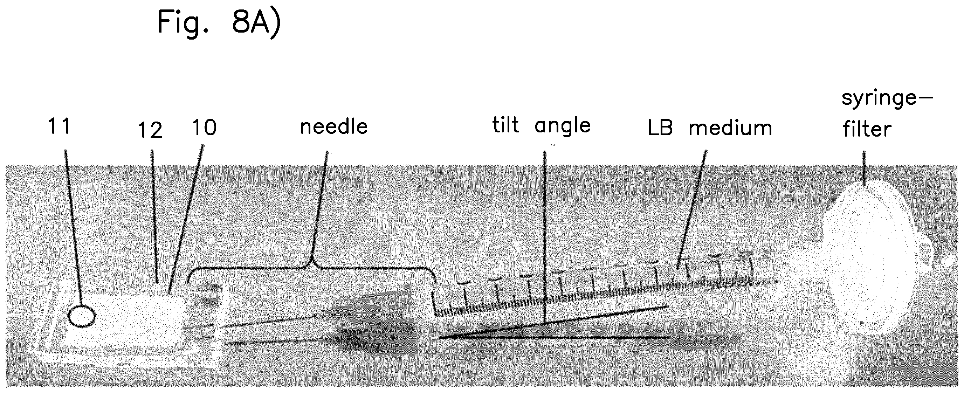

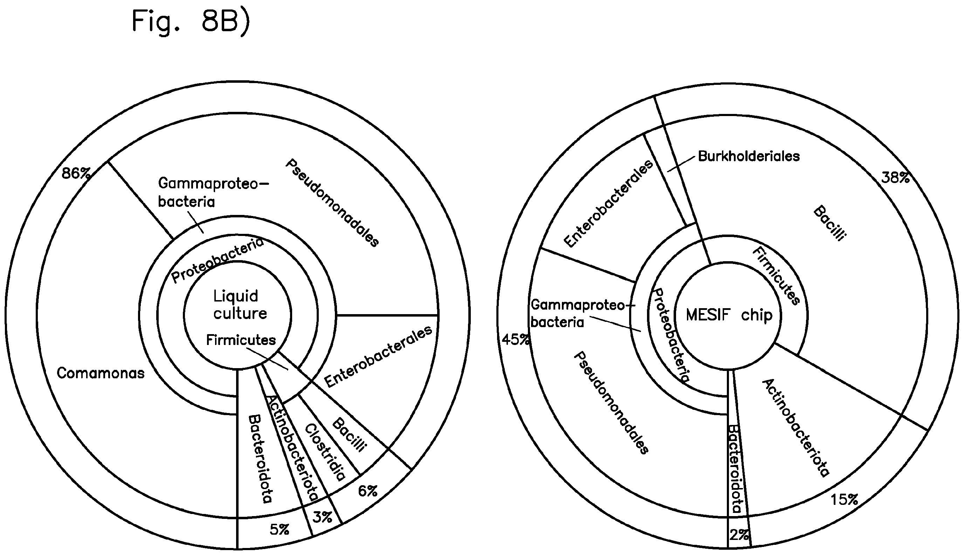

For this purpose an adapted device comprising a MESIF-chip (12) was filled with LB cultivation medium and connected from the side of the reservoir (10) to a syringe through a needle (example 4C), figure 8A)). A sterile syringe-filter was plugged into the other side of the syringe to allow for ventilation. The syringe was filled with LB medium to serve as an extended reservoir for the chip, enabling wet conditions and continued viable cultivation. Chips with the environmental contact hole (11) facing upwards were placed in an open interior of a poultry farm with a stocking density of 2 chickens per m², and incubated for several days, then collected and stored at -20 °C until further analysis. As a control, the same LB medium in an open sterile Eppendorf tube was incubated at the same conditions. Genomic DNA was extracted and 16S rRNA amplicon sequencing and bioinformatic analysis were conducted. While the comparison sample showed a complete overgrowth of *Proteobacteria* (figure 8), representing 86% of the total abundance, the MESIF-chip (12) enabled the isolation of a much more evenly distributed composition of airborne microorganisms, particularly *Proteobacteria, Firmicutes* and *Actinobacteriota.* The latter taxonomic group is particularly interesting because of its ability to produce specific secondary metabolites such as anticancer, antifungal and immunosuppressive agents. Thus, two-thirds of all known antibiotics are currently produced by *Actinobacteriota,* which are therefore of great medical and biotechnological interest (A. van der Meij et al., FEMS Microbiol. Rev. 2017, 41 (3), 392-416; D. De Simeis et al., Antibiotics 2021, 10 (5), 483). Also, the broad distribution of the different microorganisms on the MESIF-chip (12) shows very clearly that the simple and pragmatic reservoir concept presented here is very well suited to sample complex airborne habitats, even when using a non-optimal medium such as LB, which should rather favor the enrichment of well-culturable organisms. Since the MESIF and reservoir can be loaded with a variety of substances, this approach should also be applicable for targeted enrichment of microorganisms, for example, by filling the reservoir with a chemical substance that attracts or repels specific microorganisms.

The low-cost, bioinert and widely modifiable chip (12) containing at least one piece of a macroporous elastomeric silicone foam (5), according to the invention, is mechanically robust and resilient, making it applicable in a wide range of very challenging, complex habitats that include dry, wet and liquid environments, such as indoor air, soils, turbulent wet moving bed biofilters or wastewater treatment plants. The macroporous silicone foam (5), according to the invention, is, despite its bioinert surface, colonized by microorganisms surprisingly quickly and efficiently *in situ,* such that after a few days of incubation in diverse environmental habitats, a stable population is formed within the foam that can be identified using next generation sequencing. Even complex microbial communities can be established within the macroporous silicone foam (5).

The macroporous elastomeric silicone foam (5) according to the invention can be manufactured on a large scale, offers ideal wetting and diffusion properties, can be chemically tailored with respect to its surface area, and can easily be integrated into selection chips available via soft-lithographic fabrication.

Since the chip (12) containing at least one piece of a macroporous elastomeric silicone foam (5) can be easily loaded with solid, liquid and gaseous substances that influence microbial colonization, it can also be used for the targeted enrichment of microorganisms and individual inhabitants of a particular environment.

The present invention allows the systematic study of microbial dark matter and also enables new approaches for sampling, enrichment, cultivation, identification and exploitation of previously uncultivable or unknown microorganisms *in situ.*

The invention is explained in more detail with reference to the following figures, examples of embodiments and descriptions. All features shown and combinations thereof are not limited to these figures and examples of embodiments. Rather, these are to be considered representative of further possible embodiments that can be combined, but which are not explicitly shown.

### Figures:

### List of reference signs

(1) Device containing a macroporous elastomeric material
(2) Macroporous elastomeric material/macroporous matrix
(3) First donor site
(4) Second donor site
(5) Macroporous elastomeric silicone foam (MESIF)
(6) Porogen crystals
(7) Silicone prepolymer solution
(8) Casting mold
(9) PDMS housing structure
(10) Reservoir
(11) Environmental contact hole
(12) Chip containing at least one piece of a macroporous elastomeric silicone foam (5) (MESIF-chip)
(13) PDMS cover lid

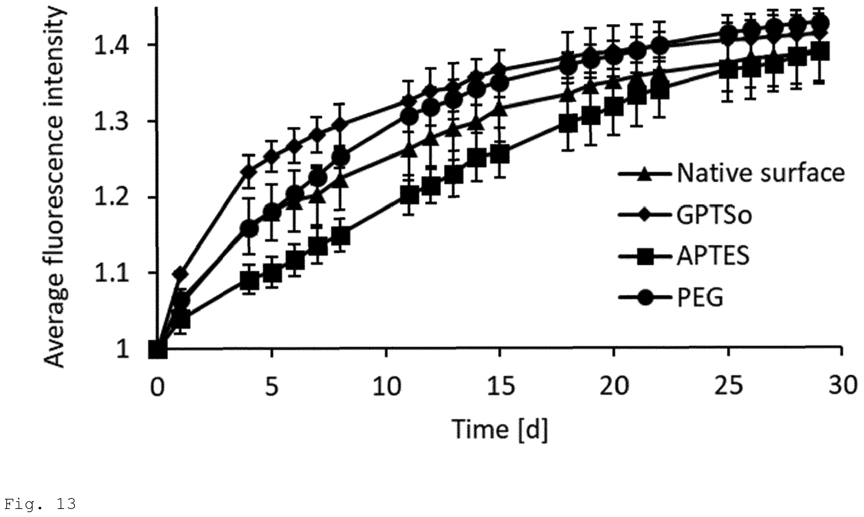

Figure 1:
   Figure 1 shows a scheme of a device (1) where a macroporous elastomeric material (2) as macroporous matrix is placed between a first donor site (3) and a second donor site (4).
Figure 2:
   Figure 2 shows an experimental setup where a macroporous elastomeric silicone foam (MESIF) (5) as macroporous matrix was placed between a first donor site (3) and a second donor site (4) (figure 2A)). For the experiment the macroporous elastomeric silicone foam (MESIF) (5) was integrated into a test tube as well as a cuvette, containing LB medium with or without agar (representing the second donor site (4)) to isolate microbes from air (representing the first donor site (3)) (figures 2B), 2C)).
Figure 3:
   Figure 3 shows the preparation of a macroporous elastomeric silicone foam (MESIF) material and the chip concept according to the invention. Figure 3A) shows the workflow of MESIF material preparation by mixing porogen crystals (6) (sodium chloride) with a PDMS prepolymer solution. The mixture is then molded and cured in a casting mold (8), followed by porogen leaching to yield a macroporous elastomeric silicone foam (MESIF) (5). Figure 3B) shows the integration of the macroporous elastomeric silicone foam material into a PDMS housing structure (9) bearing a reservoir (10) to take up the foam material and at least one environmental contact hole (hole for contact with the environment) (11). Figure 3C) shows the use of a MESIF-chip (12) for the targeted enrichment of microbial communities: A culture medium or growth substrate is injected into the reservoir (10), filling the entire MESIF (5). Insertion of the chip (12) containing at least one piece of a macroporous elastomeric silicone foam (5) (MESIF-chip) into the habitat to be sampled allows microbial communities in the isolation sample to enter the environmental contact hole (11) and to spread within the MESIF (5) towards the nutrients in the reservoir. After incubation is complete, the MESIF-chip (12) is recovered from the habitat and DNA is extracted from the MESIF (5) and subsequently sequenced.
Figure 4:
   Figure 4 shows the workflow of the MESIF-chip (12) assembly. A casting mold (8) comprising two parts (figure 4A)) is closed with screws and filled with porogen crystals (6) (sodium chloride) and a PDMS prepolymer solution (figure 4B)). After thermal curing, the mold is disassembled and the molded MESIF piece (65 x 10 x 3 mm³) (figure 4C)) is cut into pieces of approximately 18 x 10 x 3 mm³ (figure 4D)) to fit into a PDMS housing structure (9) (figure 4F)), which was fabricated using a corresponding casting mold (figure 4E)) and which is bearing a reservoir (10) to take up the MESIF material. The housing structure containing the individual MESIF pieces (figure 4G)) is then covered with a PDMS cover lid (13), which was previously molded and into which the environmental contact holes (11) were manually inserted with a press puncher. Finally, the three components of the chip, housing, MESIF, and lid, were assembled as shown in figure 4H) and sealed by bonding.
Figure 5:
   Figure 5 shows morphology and diffusion properties of MESIF materials. Figure 5A): Electron micrographs of the MESIF material. Cross section of a MESIF (5) prepared with porogen crystals (6) of >707 µm (I), 0 - 354 µm (II) size fractions and unsieved table salt (III). Open connection between two pores indicated by a white arrow (IV), with zoom-in through the opening to show the back pore (V). Close up on the surface morphology of the pores, reveals PDMS structures on the pore surface (VI). Figure 5B): Pore size distribution of MESIF materials prepared with porogen crystals (6) of variable grain size, as determined from ESEM micrographs. Figure 5C): Influence of different pore sizes on the diffusion behavior of model solute 5-carboxyfluorescein (10 µM), applied as a drop to the environmental contact hole (11) in the MESIF-chip (12). The curves, obtained by automated fluorescence image analysis as detailed in figure 5C), show the increase in fluorescence over the total area of the chip with time. Note that small pore size leads to slightly faster diffusion. Error bars are standard deviation of three independent chips. Figure 5D) shows the distribution of grain sizes as determined by sieve analysis. Shown is the percentage distribution of porogen size fractions resulting from screening crude salt crystals through a sieve with interchangeable sieve inserts. The inserts used had sizes of 25 mesh (707 µm), 35 mesh (500 µm), 45 mesh (354 µm), 60 mesh (250 µm), and 80 mesh (177 µm).
Figure 6:
   Figure 6 shows the growth of *E. coli* cells expressing the red fluorescent protein *(E. coli-RFP)* in a MESIF-chip (12). Figure 6A): Effect of different pore sizes on growth behavior. *E. coli-*RFP was inoculated into the environmental contact of the MESIF-chip (12) containing at least one piece of a macroporous elastomeric silicone foam (5) and incubated at 37 °C for several days. The time course of growth was determined by the increase in fluorescence intensity over the entire area of the chip. Note that larger pore sizes result in a slightly increased spreading velocity of cells in the MESIF. Figure 6B): Influence of the surface modification of MESIF prepared with 500 - 707 µm porogen crystals (6) on the growth behavior. Note that the presence of PEG groups appears to promote faster spreading of E. *coli-RFP* inside the chip. Error bars in (A) and (B) are standard deviation of three independent chips.
Figure 7:
   Figure 7 shows the phylogenetic distribution of microorganisms in a moving bed biofilter of a fish farm. Shown are KRONA charts of a 16S rRNA amplicon analysis of microorganisms that grew on a commercial biofilm support (Mutag Biochip^{™}) (figure 7A)) or on MESIF (figure 7B)). Both carriers were incubated for 18 days in the biofilter of a commercial aquaculture facility. The MESIF (figure 7B)) shows a significantly higher biodiversity than the Mutag Biochip^{™}.
Figure 8:
   Figure 8 shows the phylogenetic distribution of airborne microorganisms from a poultry farm environment. Figure 8A): The reservoir (10) of the MESIF-chip (12) was connected via a needle (liquid-liquid connection) with a syringe containing Luria Broth (LB) medium for continuous supply of sterile cultivation medium, the syringe being plugged with a syringe-filter (sterile vent) and having a minimum tilt angle to the surface to provide a continuous supply of LB medium into the MESIF-chip (12). Figures 8B), 8C): KRONA charts of the 16S rRNA amplicon sequencing analysis of microorganisms that were allowed to grow for 9 days in open control tubes filled with LB media (figure 8B)) or the MESIF-chip (12) (figure 8C)). Note that the MESIF-chip-based sampling led to a higher biodiversity and was able to enrich the biotechnological relevant group of *Actinobacteriota* by 5-fold as compared to the liquid medium.
Figure 9:

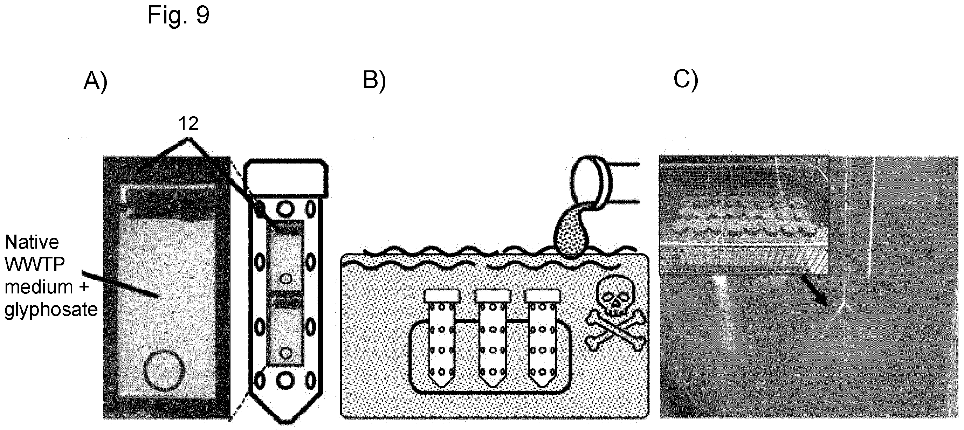

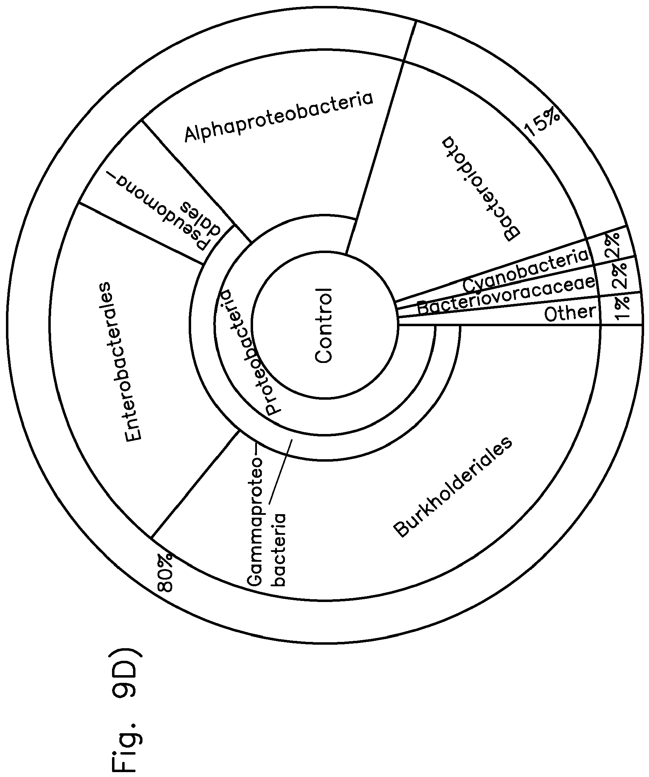

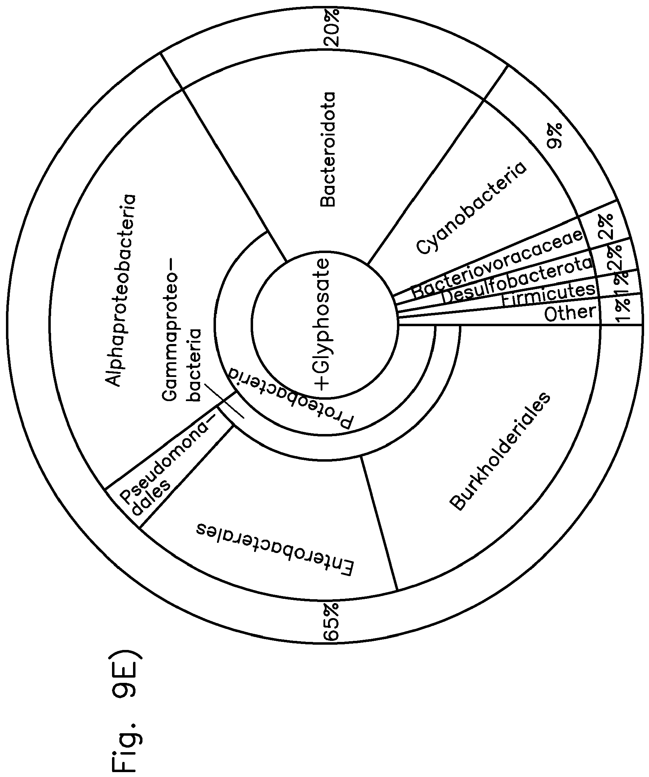

   Figure 9 shows the targeted enrichment and sampling of microorganisms from a chemical wastewater treatment plant (WWTP) under glyphosate selection. The MESIF-chip's reservoir (10) was filled with a sterilized native wastewater treatment plant (WWTP) medium supplemented with glyphosate (8 g/L) or pure sterilized WWTP medium as control. Figure 9A): Two chips were packed into a 50 mL Falcon tube equipped with handmade openings to allow inflow of WWTP effluent. The Falcon tubes were fixed in a metal rack and immersed ~40 cm deep in the static chemical wastewater collection tank in the open air (figures 9B), 9C)) and left for six days. Figures 9D), 9E): KRONA charts of the 16S amplicon sequencing analysis of microorganisms that were collected from the control (figure 9D)) or the MESIF-chip (12) (figure 9E)). Note that the glyphosate-based sampling led to an enrichment of *Desulfobacteria, Firmicutes* as well as a substantial larger fraction of *Cyanobacteria.*
Figure 10:
   Figure 10 shows the structural formulae of the surface modification applied to MESIF materials. Figures 10A) and 10D) show MESIF surfaces modified with 2% (v/v) APTES ((3-Aminopropyl) triethoxysilane) after surface activation with HCl_{(aq)} and H₂O₂, figures 10B) and 10E) show MESIF surfaces modified with 2% (v/v) GPTS (3-Glycidyloxypropyl) trimethoxysilane) (I), where the epoxide rings are subsequently opened by acid hydrolysis (II), and figures 10C) and 10F) show surfaces modified with PEG (Polyethylene glycol).
Figure 11:
   Figure 11 shows the installation of DNA oligonucleotides on the surface of the macroporous elastomeric silicone foam (MESIF) (5). Epoxy-activated MESIF, prepared with GPTS as described in figure 10B), was coupled with DNA oligonucleotides AmC12Tr12-Cy5 (red, λExc = 633 nm, λEm = 666 nm) (figure 11B)), followed by hybridization with the complementary DNA oligomer Cy3-cTr12 (green, λExc = 561 nm, λEm = 568 nm) (figure 11C)). The resulting double helix AmC12Tr12-Cy5 - Cy3-cTr12 can be detected by FRET signals. GPTS-modified MESIF with opened epoxy groups was used as control.
Figure 12:

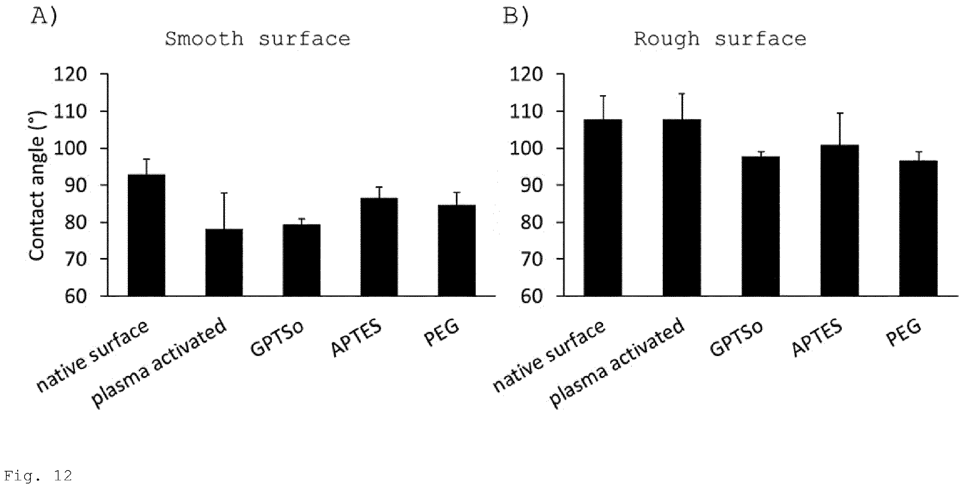

   Figure 12 shows diagrams of water contact angle measurements to confirm the successful chemical modification of the MESIF surfaces. A 7 µL deionized H₂O droplet was applied either to the smooth cast surface of a MESIF (5) that was in contact with the PMMA mold during casting (figure 12A)), or to a rough, porous surface of freshly scalpel-cut MESIF material (figure 12B)). The angle between the MESIF material surface and the water droplet was recorded with a camera, and the contact angle was quantified with ImageJ. The various MESIF surface modifications were prepared as described in example 3 and figure 10. The smooth cast surface of a MESIF that was in contact with the PMMA mold during casting (figure 12A)) and the rough, porous surface of freshly scalpel-cut MESIF material (figure 12B)) were compared with different MESIF-modifications, achieved with 2% (v/v) APTES, 2% (v/v) GPTS and 0.25% (w/w) PEG modified MESIF materials, respectively.
Figure 13:
   The diagram in figure 13 shows the influence of different surface modifications on the diffusion behavior of 5-carboxyfluorescein in MESIF-chips (12), prepared with porogen crystals (6) of 500 - 707 µm grain size. Compared MESIF materials were either non-modified (native), modified with epoxide ring opened GPTS, APTES, PEG block copolymer, as detailed in figure 10. The model solute 5-carboxyfluorescein (10 µM) dye was applied to the environmental contact hole (11) in the MESIF-chip (12) and the course of diffusion over time was determined as described in figure 5C). Error bars are standard deviation of three independent chips.

### Examples:

### Example 1:

Fabrication of a macroporous elastomeric silicone foam (MESIF) (5) and a chip (12) containing at least one piece of a macroporous elastomeric silicone foam (5) (MESIF-chip).

### MESIF production:

Initially, a casting mold (8) comprising two parts (figures 3A) and 4A)) was designed using Autodesk Inventor software 2020 (Autodesk Inc., USA) and milled with a CNC micromilling machine (Mini Mill GX, Minitech Machinery, USA). The two parts of the mold were closed by 9 screws. As porogen crystals (6) was used sodium chloride table salt (ChanteSel iodized salt fine from Lidl, Germany), the particles of which were fractionated using a sieve with the following inserts: 25 mesh (707 µm), 35 mesh (500 µm), 45 mesh (354 µm), 60 mesh (250 µm) and 80 mesh (177 µm). The resultant porogen fractions were weighed individually and the ratio to the mass of the sieved salt was calculated to estimate the mass fractions. To prepare a PDMS prepolymer solution, PDMS oligomers were mixed with the curing agent (Sylgard 184, Down Corning) in a ratio of 10:1, respectively, then degassed in a vacuum chamber for 1 hour. 0.9 mL of the PDMS prepolymer solution were poured inside the closed casting mold (8) to fill approximately half of its volume and the remaining free volume was filled with table salt porogen crystals of the desired particle size. The filled mold was centrifuged (5804 R, Eppendorf) at 4600 rpm and 4 °C for 20 minutes to disperse and pack the porogen crystals (6) within the liquid PDMS prepolymer. After centrifugation, the PDMS-porogen mixture was cured in the oven (Heraeus, Thermo Scientific Inc.) at 70 °C for one hour, then the mold screws were removed and the solid, casted PDMS-porogen mixture piece was carefully released from the mold as a single piece (~65 × 10 × 3 mm³) as shown in figure 4C). To leach the porogen from the cured PDMS-porogen, the released piece was repeatedly pressed by hand in a 50 °C water bath until the material reached a soft, elastic, and spongelike structure. To remove all salt residues, the material was stirred overnight in a beaker filled with 250 mL ddH₂O at 40 °C and washed in the next morning with fresh ddH₂O. The resultant MESIF material was then dried at 90 °C for one hour in the oven, then cut into pieces of ~18 × 10 × 3 mm³ (figure 4D)) using a scalpel. These final MESIF pieces fit into the MESIF-chip PDMS housing structure (9) (figures 4F) and 4G)).

### MESIF-chip (12) production:

To produce a MESIF-chip (12), a casting mold corresponding to the PDMS housing structure (9) was designed using Autodesk Inventor software and milled on a PMMA plate using a CNC micromilling machine. To cast the PDMS housing structure (9), a PDMS prepolymer solution was prepared as described above and poured into the chip mold, then cured at 70 °C for one hour in an oven. After curing, the cast was peeled off the mold and was ready to fit the MESIF material pieces inside (figure 4E)). Likewise, a mold for the PDMS cover lid (13) (76 × 26 × 0.75 mm³) was prepared using the same procedure, into which the environmental contact holes (11) (ø 4 mm) were manually inserted with a press puncher. For assembling the MESIF-chip (12), the prepared three components (MESIF pieces, PDMS housing structure (9) and PDMS cover lid (13)) were individually activated by plasma treatment (Plasma Flecto10, PlasmaTechnology) for 30 seconds at 300 W and 0.2 mbar. Then, the MESIF pieces were placed in the corresponding empty space in the PDMS housing structure (9), covered with the PDMS cover lid (13) (figure 4H)) and the assembled chip was cured at 90 °C for 30 min for permanent bonding.

### Example 2:

Physical characterization of the macroporous elastomeric silicone foam (5):
Material and porogen crystals morphology:
To gain insight into the physical properties of the MESIF materials, the grain sizes of the porogen crystals (6) used, commercial table salt (ChanteSel Jodsalz fein from Lidl stores, Germany) was analyzed. For this purpose, the distribution of grain sizes was determined by dividing the salt into the respective grain sizes using a sieve with interchangeable sieve trays. The inserts used had pore sizes of 707 µm, 500 µm, 354 µm, 250 µm and 177 µm. The masses of the respective size fractions of the sieve analysis and the percentage share of the total mass of the sieved salt were determined (figure 5D)). The fractions 707 - 500 µm and 500 - 354 µm showed the largest mass fraction with together almost 80%. Sufficient amounts of salt crystals were obtained from the >707 µm, 707 - 500 µm, 500 - 354 µm and 354 - 250 µm salt fractions to prepare materials and perform further analysis of MESIF pore sizes, diffusion and growth behavior with model organisms.

To visualize the pore structure and morphology within the MESIF material, the MESIF material was cut into approximately 1 mm thick layers with a scalpel, then coated with 1.5 nm platinum and examined using Environmental Scanning Electron Microscopy (ESEM) at 20 KV under vacuum. The software ImageJ was used for further analysis of the acquired images. By setting a gray threshold and manual post-processing, all pores were colored black and separated from each other and evaluated using ImageJ's "Analyze Particle" function.

Figure 5A) shows representative ESEM images of MESIF prepared with different porogen grain sizes. The images show a stochastic arrangement of pores characterized by different shapes reflecting the morphology of salt crystals. At high magnification, many pores show sharp-edged cracks in the pore surface at their contact surfaces (figure 5A) IV) through which the pore behind can be seen when focused further (figure 5A) V). Also, thin, crumpled PDMS pieces are found in some pores (figure 5A) VI). These optical findings suggest that the interconnecting cracks between individual pores are formed in the course of porogen leaching, resulting in interconnectivity between the pores. The ESEM images were also used to determine the pore size distribution of the two-dimensional pore cross sections of the sponges using ImageJ analysis (C. A. Schneider et al., Nat. Methods 2012, 9 , 671-675) (figure 5B)). The histograms show absolute pore sizes smaller than experimentally determined porogen particle sizes, because only a two-dimensional section of the three-dimensional MESIF material was recorded in the ESEM and thus the pores were not imaged in their maximum cross-sectional area depending on the position of the section. Despite this systematic error, it is visible that with larger salt crystals the pore size distribution becomes progressively wider and flatter. In contrast, smaller salt crystals produce narrower and steeper distributions. The pore size distribution of the unsieved salt shows a superposition of the distributions of all size fractions.

### Diffusion experiment:

The homogeneous interconnectivity of the pores as well as the diffusion behavior within the chip (12) containing at least one piece of a macroporous elastomeric silicone foam (5) were investigated with the help of the fluorescent model solute 5-carboxyfluorescein (λExc = 492 nm, λEm = 517 nm) (figure 5C)).

On the one hand, this method revealed that the fluorophore spreads almost homogeneously throughout the foam. On the other hand, the method could be used to quantify differences of the foam on the dissociation of the solute and also of fluorescently labeled model organisms. As an example, it is shown in figure 5C) that the pore sizes of the MESIF have little effect on the passive diffusion of the fluorescent solute. However, the trend was evident that smaller pore sizes lead to faster diffusion, presumably due to higher capillary forces in the smaller pores.

A stock solution of the dye (100 mM in dimethyl sulfoxide) was stored in a -20 °C freezer, and a dilution of 10 µM in ddH₂O was freshly prepared upon use. Chips with different MESIF pore sizes were filled with ddH₂O, which was injected into the chip reservoir with a syringe and perfused through the MESIF until it exited the environmental contact hole (11). At least 1 mL of ddH₂O was injected into the chip to ensure that the MESIF was completely soaked. The filled chips were then placed in a Petri dish with the environmental contact holes (11) facing upwards, then 40 µL of 10 µM 5-carboxyfluorescein solution was loaded onto the contact hole. 5 mL ddH₂O were added to the Petri dish to ensure adequate humidity. The dish lid was closed and sealed with parafilm to prevent the chips from drying. The Petri dish was kept at room temperature in the dark for the entire experiment to avoid photobleaching. To determine the diffusion progress, images of the Petri dish were taken over the experimental time using a fluorescence gel imager (Fusion FX, Vilber) at exposure time of 1 ms. At least three independent chips were used in these experiments. For microbial growth in the MESIF-chip, a lab stock *E. coli* DH5α strain (Groesche et al., Small 2019, 15, 1901956) transformed with a plasmid bearing kanamycin selection marker gene and arabinose inducible red fluorescent protein (*E*. *coli-RFP*) was used. A pre-culture of 5 mL LB medium (10 g/L tryptone, 5 g/L yeast extract, 10 g/L NaCl, all dissolved in ddH₂O and autoclaved), supplemented with 50 µg/mL kanamycin and 10 mM arabinose was inoculated with *E. coli-*RFP and incubated overnight at 37 °C in 180 rpm shaker. 20 µL of this pre-culture were loaded onto the contact hole of MESIF-chips, previously autoclaved at 121 °C for 20 min and prefilled with LB medium containing 50 µg/mL kanamycin and 10 mM arabinose, using the same method explained above. The inoculated chips were then placed in a sterile Petri dish, and 5 mL ddH₂O were added to the Petri dish to ensure humidity. The Petri dish was sealed with parafilm and incubated at 37 °C for the respective incubation times. Growth progress indicated by red fluorescence was documented using the gel imager. At least three independent chips were used.

### Example 3:

### Chemical surface modification:

To activate the surface of the MESIF materials, the protocol developed by Sui et *al.* was used (G. Sui et al., Anal. Chem. 2006, 78, 15, 5543-5551). In brief, MESIF materials were immersed in the activation solution composed of ddH₂O, concentrate H₂O₂ (30% v/v) and concentrate HCl (37% v/v) in a volume ratio of 5:1:1 at room temperature. In the activating solution, the materials were mechanically squeezed by hand for 2 min, followed by stirring in the same solution for 3 min. This procedure was repeated a total of five times. Afterwards the MESIF materials were washed with ddH₂O until a neutral pH value in the washing water was achieved. Finally, the materials were dried overnight in an oven at 90 °C.

Three different surface functionalization methods were used to install amino-, epoxy- or polyethylenglycol groups on the MESIF surface using, (3-Aminopropyl) triethoxysilane (APTES, SigmaAldrich) (figure 10A)), (3-Glycidyloxypropyl) trimethoxysilane (GPTS, SigmaAldrich) (figure 10B)) or a PDMS-PEG block copolymer (figure 10C)). Modification with APTES and GPTS (figures 10D) and 10E)) was achieved by following the method of Yu et *al.* (Q. Yu et al., Colloids Surf., B, 2010, 76, 468-474). In brief, the surface activated MESIF materials were heated in a solution of toluene containing the appropriate organosilane (2% v/v) at 80 °C for 24 h to obtain the desired surface functionalization. The materials were then washed with toluene and dichloromethane and any solvent residues were removed at 40 °C and under reduced pressure.

To open the epoxy ring of the GPTS (figure 10E)), GTPS-functionalized MESIF materials were immersed in a 1:1 mixture of concentrate HCl (37% v/v) and ddH₂O. The materials were mechanically squeezed by hand for 25 min in the acidic solution in order to open the epoxide to the corresponding diol. Afterwards the MESIF pieces were washed with ddH₂O until a neutral pH value was reached in the washing solution. The pieces were then dried overnight in an oven at 90 °C.

### Functionalization with PEG copolymer:

PDMS oligomers and a thermal curing agent were mixed at a ratio of 9:1 as a polymerization mixture. Additionally, a PDMS-PEG block copolymer (Gelest Inc.) was added in a ratio of 0.25 % (w/w), to introduce PEG-units into the material (figure 10F)). The resulting PDMS prepolymer solution was then used to prepare MESIF materials as described in example 1.

### Coupling of DNA oligonucleotides to GPTS-functionalized MESIF:

To couple DNA oligonucleotides to the surface of GPTS-functionalized MESIF (figure 11), MESIF pieces were incubated in 3 mL of phosphate buffered salt solution (PBS) containing 100 nm of the DNA oligonucleotide AmC12Tr12-Cy5 (5'-Amine-C12-T8-(ATG)4-Cy5-3 The MESIF pieces were shaken in the solution for 6 h, at room temperature and at 175 rpm. To improve mass transport inside the MESIF, the pieces were squeezed manually with a glass rod every 0.5 h for 1 min. Following, the MESIF pieces were washed five times with PBS, each time the MESIF materials were squeezed with a glass rod for 1 min. Removal of unbound DNA was monitored by UV/Vis absorbance measurements of the washing solution. For hybridization of the DNA-functionalized MESIF, the pieces were incubated in 3 mL of PBS containing 100 nM of the complementary oligomer Cy3-cTr12 (5'-Cy3-(CAT)4-3'). Incubation was done under shaking for 6 hours at room temperature at 175 rpm and repeated squeezing every 0.5 h with a glass rod. The pieces were then washed five times with PBS, and the UV/Vis absorbance of the washing solution was measured. The so-treated MESIF was then examined to accurately localize DNA within the pore structure, using confocal laser scanning microscopy (CLSM, ZEISS LSM 880, Carl Zeiss Inc.).

Excitation wavelengths and filters for Cy5 (λExc = 633 nm, λEm = 666 nm), Cy3 (λExc = 561 nm, λEm = 568 nm) and by transmitted light were used. The successful hybridization and thus formation of double-stranded DNA was confirmed via Forster Resonance Energy Transfer (FRET) with Cy5-excitation and measured with Cy3-emission (λExc = 561 nm, λEm = 666 nm). This analysis was applied to DNA-modified MESIF before and after hybridization.

### Example 4:

### Example 4A) Microbial growth and sampling studies:

The growth of model bacteria in MESIF materials was investigated by using genetically modified *Escherichia coli* cells expressing intracellularly the red fluorescent protein (*E. coli-RFP*). To obtain quantitative data, growth experiments were performed in MESIF-chips (12) with different pore sizes as well as different surface modifications, analogous to the diffusion experiments described in example 2 and figure 5. For this purpose, 20 µL of *E. coli-RFP* preculture (OD₆₀₀ ~ 3) in LB medium were pipetted onto the contact hole of the chips, which were previously saturated with LB medium. The inoculated chips were placed in Petri dishes, 5 mL of water were added, and the dishes were sealed with Parafilm and incubated at 37 °C for up to 22 days. The propagation of the fluorescence signal over the entire chip area was documented by regular imaging. After only two days, the growth of fluorescent bacteria was visible to the naked eye as a pink coloration of the material. Confocal microscopy imaging revealed the MESIF microstructure, which showed three-dimensional interconnected pores filled with E. *coli-RFP.*

Magnified micrographs inside a pore showed cells growing in dense populations and biofilms. Quantitative assessment of the E. *coli-RFP* growth in the MESIF-chips (12) revealed that larger pore sizes led to a slightly faster cell migration inside the MESIF (figure 6A)). The influence of MESIF surface modifications on the growth behavior of E. *coli-RFP* was also investigated (figure 6B)). Triplicate measurements, similar to the investigation of the influence of pore sizes (figure 6A)), suggested that modification with PEG groups led to faster spreading of the *E. coli* cells within the MESIF. Although the fluorescence-based growth observations are very useful for quantitative assessment under laboratory conditions, this approach is unsuitable for realworld applications in the field because microorganisms rarely exhibit strong autofluorescence signals and often display very slow reproduction times. MESIF materials also show altered turbidity and opacity due to microbial colonization, which can be detected very easily by UV transillumination. Using the example of the very slow growing *Bacillus merisflavi,* the bacterial growth was monitored in real time with a normal webcam and used to quantify the biomass in the MESIF-chip (12).

### Example 4B) Sampling of microorganisms from moving bed biofilter:

To test the suitability of the macroporous elastomeric silicone foam (5) for enriching complex microbial communities from natural habitats, an aerobic moving bed biofilter of a fish farm was chosen where pikeperch are kept at a stocking density of approx. 15 kg/m³. To evaluate the performance of the MESIF material, commercially available polyethylene biofilter carrier chips (Mutag Biochip^{™}) were used as reference material. These chips are reported by the manufacturer to have a high protected active surface area of more than 5,500 m²/m³, and are often used as biocarriers for moving bed biofilm reactor studies (N. H. Dan et *al.,* Sci. Total Environ. 2021, 787, 147680; A. A. Mazioti et *al.,* Water 2021, 13, 1810). MESIF pieces (10 × 10 × 3 mm³) prepared with unsieved porogen crystals and Mutag chips (Mutag Biochip^{™}) (ø 300 mm) were sterilized by autoclaving 121 °C for 20 min. Sterile MESIF-chips (12) and Mutag chips (Mutag Biochip^{™}) were then incubated freefloating in the biofilter tank for 18 days, then collected, briefly washed with ddH₂O, and frozen at -20 °C for temporary storage and transport. Duplicate MESIF-chips and Mutag chips (Mutag Biochip^{™}) were used.

Genomic DNA (gDNA) was extracted from the chips using commercially available extraction kits according to the manufacturer's protocol, and the amount of extracted gDNA was quantified photometrically. Characterization of the obtained gDNA was done by gel electrophoresis and 16S rRNA amplicon sequencing. The quality of the extracted gDNA was sufficiently high to produce a sequencing library for Illumina sequencing using commercially available kits. Following Illumina 16S rRNA amplicon sequencing, the sequences found were bioinformatically processed, analyzed, and visualized as Krona charts. The resulting KRONA charts in figure 7 illustrate that the microbial community in the Mutag Biochip^{™} is strongly dominated by *Alphaproteobacteria* and *Planctomycetes* (figure 7A)), the latter being especially known to form biofilms on various surfaces (S. Wiegand et al., Nat Microbiol 2020, 5 (1), 126-140). In contrast, the MESIF-chip (12) showed a much broader distribution of different bacterial species (figure 7B)). Notably, in the MESIF-chip (12), species from the so called *Candidate Phyla Radiation* (CPR) (S. Wiegand et al., Front. Microbio. 2021, 12, 635506) could be enriched.

### Example 4C) Sampling of airborne microorganisms:

To test real-life applicability of MESIF-chips for environmental issues, sampling of airborne microbial communities was investigated. For this purpose, the designed reservoir feature of the MESIF-chip (12) was exploited to provide a continuous supply of a LB medium to the porous interior of the chip to enable for a long incubation period in a dry environment. The adapted device comprised a MESIF-chip (12) filled with LB medium and was connected from the side of the reservoir (10) to an 1 mL syringe through a needle (both from BBraun GmbH) (figure 8A)).

A 0.2 µm sterile syringe-filter was plugged into the other side of the syringe to allow for ventilation. The syringe was filled with LB medium to serve as an extended reservoir for the chip, enabling wet conditions and continued viable cultivation.

In this simple setup, the syringe serves as a sterile aerated fluid reservoir from which the loss of moisture in the MESIF (5) - due to evaporation at the environmental contact - is continuously replenished as the foam acts as a capillary pump.

Chips with the environmental contact hole (11) facing upwards were placed on top of a 2 m high cabinet in an open interior of a poultry farm with a stocking density of 2 chickens per m², and incubated for nine days, then collected and stored at -20 °C until further analysis. As a control, 1 mL of the same LB medium in an open 1.5 mL sterile Eppendorf tubes were incubated at the same conditions. Triplicate MESIF-chips (12) and control Eppendorf tubes were used.

Genomic DNA was extracted and 16S rRNA amplicon sequencing and bioinformatic analysis were conducted as described above. While the liquid culture showed a complete overgrowth of *Proteobacteria* (figure 8B) left), representing 86% of the total abundance, the MESIF-chip enabled the isolation of a much more evenly distributed composition of airborne microorganisms, particularly *Proteobacteria, Firmicutes* and *Actinobacteriota* (figure 8B) right).

Example 4D) Sampling of microorganisms from chemical wastewater: To demonstrate that the MESIF-chip is applicable for selective enrichment of microbial communities when the reservoir is filled with a specific chemical, sampling studies were conducted in the highly challenging wet habitat of a wastewater treatment plant (WWTP). For this purpose, the possible selective enrichment of microorganisms was tested by using the herbicide glyphosate, which is a phosphonic acid compound and is used as the biologically active major component of some broad-spectrum or total herbicides on a millionton scale worldwide (M. E. Richmond, J Environ Stud Sci 2018, 8 (4), 416-434).

MESIF-chips (14) were prepared with unsieved porogen salt and autoclaved. To enable sampling, the reservoir of the MESIF-chip (12) was filled with a sterilized native WWTP medium supplemented with 8 g/L glyphosate (Monsanto, Bayer). Chips whose reservoirs were filled with sterilized WWTP medium only were used as controls. Two sterile chips loaded with either glyphosate or the control medium were packed into a 50 mL Falcon tube, in which handmade openings allow inflow of WWTP effluent (figure 9A)). The Falcon tubes loaded in this manner were fixed in a metal rack (figure 9B)) and then immersed ~40 cm deep in a static chemical wastewater collection tank of the waste management system of the Karlsruhe Institute of Technology in the open air (figure 9C)) and left for six days. The chips were then collected and gently washed with sterile ddH₂O followed by storage at 20 °C until further analysis. Duplicate MESIF-chips (12) with or without glyphosate were used. 16S rRNA amplicon sequencing and bioinformatic analysis were conducted afterwards.

The Krona chart (figure 9D)) shows that the MESIF control chip lacking glyphosate showed mainly different *Proteobacteria,* as well as some *Bacteroidota* and a small amount of *Cyanobacteria* and *Bacteriovoracaceae.* In contrast, the glyphosate-containing MESIF-chip showed an almost 5-fold larger fraction of *Cyanobacteria,* suggesting their enrichment due to the presence of glyphosate (figure 9E)). Furthermore, the *phyla Desulfobacterota* and *Firmicutes* could be found in the glyphosate MESIF-chip. The occurrence of *Desulfobacterota* is especially interesting, since anaerobic sulfate or sulfur-reducing bacteria in this phylum have been described to utilize organic acids (D. W. Waite et al., Int. J. Syst. Evol. Microbiol. 2020, 70 (11), 5972-6016), which could be a metabolic product of the glyphosate degradation of other bacteria in the sample. The first steps in the degradation of glyphosate in bacteria involve the conversion of glyphosate into the weak organic acid aminomethylphosphonic acid through the glyphosate oxidoreductase (S. Singh et al., Int. J. Environ. Res. Public Health 2020, 17 (20), 7519). The acid can then be excreted into the environment or cleaved into other molecules. The presence of *Desulfo-bacterota* in the MESIF-chip containing glyphosate could thus indicate that the resulting aminomethylphosphonic acid of the herbicide degradation may be consumed by the *Desulfobacterota.* Furthermore the data regarding the presence of known glyphosate degrading organisms were analyzed by aligning all amplicon sequences from the chip against the total genomes of three organisms known for their glyphosate degradation: *Enterobacter sp. E20* (G. Cao et al., Proceedings of the International Symposium on Agriculture, Food and Biotechnology 2019 (ISAFB 2019), *Pseudomonas azotoformans* (V. Korkmaz et al., Environ. Technol. Innov. 2021, 22, 101535) and *Comamonas odontotermitis* (S. Firdous et al., Pedosphere 2020, 30 (5), 618-627). Only in case of the glyphosate containing MESIF-chip, more than 100 amplicons could be matched to different sequences within the 16S rRNA genomes of the three mentioned organisms. The same procedure performed for the amplicons from the MESIF-chip lacking glyphosate, amplicons could only be matched to sequences that were found for *Enterobacter sp. E20,* but not for the other two organisms. These results are a strong indication that the MESIF-chip with its reservoir is a very well suited platform for the targeted enrichment of microorganisms.

### Example 4E) Sequencing and bioinformatic evaluation:

Genomic DNA (gDNA) was extracted from the sampling MESIF-chips (12) described above. Under sterile condition, the MESIF (5) was removed from the chip, then was cut into small pieces using a sterile scalpel and collected in a 2 mL sterile Eppendorf tube.

The commercial Quick-DNA Miniprep Plus Kit (Zymo Research GmbH, Germany) was used to extract the gDNA from the MESIF according to the manufacturer's protocol. The concentration of extracted gDNA was subsequently quantified photometrically by NanoDrop OneC Microvolume, and fluorometrically by Qubit 3 (both from Thermo Scientific Inc.). From the purified gDNA, the 16S rRNA V3 region was amplified using the degenerate primers 341bf (ACACTCTTTCCCTACACGACGCTCTTCCGATCTCCTACGGGNGGCWGCAG) and 518r (GACTGGAGTTCAGACGTGTGCTCTTCCGATCTWTTACCGCRGCTGCTGG), which include Illumina TruSeq adapter sequences. The PCR reactions contained 0.04 U/µL High-Fidelity Q5 DNA Polymerase, 1x Q5 Reaction Buffer, 1x High GC Enhancer, 0.25 µM of each 17 primer, 0.2 mM dNTPs, and 100 ng template DNA in a total reaction volume of 25 µL. Amplification was carried out using a touchdown protocol with the following cycling conditions (95 °C, 2 min; 4 × [95 °C, 9 s; 56-52 °C, 40 s; 72 °C, 40 s] 25× [95 °C, 40 s; 51 °C, 40 s; 72 °C, 40 s]; 72 °C, 5 min; hold at 8 °C). Indexed sequencing libraries were constructed from the purified product using the NEBNext Ultra II FS DNA Library Prep Kit (New England BioLabs, Germany), using the protocol for input amounts ≥100 ng and beginning with the size selection step. Unique dual indices (NEB) were added in 6 cycles of an index-PCR. DNA library quality was verified using the Agilent High Sensitivity DNA Kit on the Agilent 2100 Bioanalyzer instrument (Agilent Technologies, Germany). Paired-end reads were generated on an Illumina NextSeq 550 (Illumina, USA) with the NextSeq 500/550 High Output Kit v2.5 (300 Cycles) (Illumina, USA). Adapter sequences and low-quality reads were removed using Trimmomatic, version 0.39, with the ILLUMINACLIP (2:30:10), SLIDINGWINDOW (4:15), MINLEN (80), and LEADING and TRAILING (both 3) options (A. M. Bolger et al., B., Bioinformatics 2014, 30 (15), 2114-2120). The remaining reads were further trimmed with BBDuk, version 38.73 (ktrim=r, mink=11, minlength=80, entropy=0.25), and Cutadapt, version 1.18 (-a 'AGATCGG$', -a 'CCGATCT$') (M. Martin, EMBnet.journal 2011, 17 (1), 10-12; B. Bushnell, BBMap - https://sourceforge.net/ projects/bbmap/ (accessed June 2022)). The high-quality paired-end reads were merged using FLASH, version 1.2.11 (15 ≤ overlap ≤ 100, maximum 5% mismatches) (T. Magoc et al., Bioinformatics 2011, 27 (21), 2957-2963) . OTU tables were compiled in four steps with the program vsearch, version 2.17.1 (T. Rognes et al., PeerJ. 2016, 4, e2584). First, the reads were dereplicated (-derep_fulllength) and singletons were removed (--minuniquesize 2). They were then clustered at 97 % identity and the cluster centroid sequences were reported. Chimeras were detected and removed with the --uchime_denovo option, and finally, reads were mapped back onto the centroids via the -usearch_global option and output as OTU tables. The centroid sequences were aligned to the SILVA SSU reference database for taxonomic classification (E. Pruesse et al., Bioinformatics 2012, 28 (14), 1823-1829). The results were visualized as a KRONA chart (B. D. Ondov et al., BMC Bioinf. 2011, 12, 385).

## Claims

1. Device (1) for the enrichment and cultivation of microorganisms *in situ* comprising a macroporous elastomeric material (2) connected to a first donor site (3) and a second donor site (4), wherein the first donor site (3) provides contact to an aqueous, solid or gaseous phase containing microorganisms, and the second donor site (4) provides contact to an aqueous, solid or gaseous phase.

2. Device (1) according to claim 1 wherein the second donor site (4) contains nutrients, a culture medium, attractants, repellents and/or other molecular as well as cellular interaction partners for the microorganisms to be collected and enriched from the first donor site (3).

3. Device (1) according to one of claims 1-2, wherein the macroporous elastomeric material (2) has interconnected pores of an average pore size ranging from 0.1 - 950 µm.

4. Device (1) according to one of claims 1-3 wherein the macroporous elastomeric material (2) is a macroporous elastomeric silicone foam (5).

5. Device (1) according to claim 4 wherein the silicone material in the macroporous elastomeric silicone foam (5) is Polydimethylsiloxane (PDMS).

6. Device (1) according to one of claims 4-5, wherein the surface of the macroporous elastomeric silicone foam (5) is chemically modified with primary amino groups, expoxy groups, polyethylenglycol (PEG) moieties, oligonucleotides or proteins.

7. Device (1) according to one of claims 4-6, wherein the macroporous elastomeric silicone foam (5) contains other materials beside silicone selected from other hydrophobic or hydrophilic polymers or blockcopolymers, and/or wherein particles made of polymers, metals, oxides or minerals are added.

8. Device (1) according to one of claims 1-7, wherein the device is a functional interface in bioreactors, tubular reactors or flat-bed reactors.

9. Device (1) according to one of claims 1-7, wherein the device (1) is a chip comprising a surface suitable for contact with the environment as the first donor site (3) and a reservoir as the second donor site (4).

10. Chip (12) containing at least one piece of a macroporous elastomeric silicone foam (5), according to claim 9, comprising a PDMS housing structure (9), a reservoir (10) holding at least one piece of a macroporous elastomeric silicone foam (5) and a PDMS cover lid (13), into which environmental contact holes (11) are punched.

11. Manufacturing process for a macroporous elastomeric silicone foam (5), comprising the following steps:
A) Mixing porogen crystals (6) with a silicone prepolymer solution (7);
B) Pouring the resulting suspension of step A) into a casting mold (8);
C) Centrifugation of the filled mold of step B) to disperse and pack the porogen crystals (6) within the liquid silicone prepolymer;
D) Thermal curing of the suspension of step C);
E) Releasing the cured macroporous elastomeric silicone foam from the casting mold (8);
F) Leaching the macroporous elastomeric silicone foam from step E) to dissolve the integrated porogen crystals (6);
G) Drying the molded macroporous elastomeric silicone foam.
H) Optionally modifying the surface of the macroporous elastomeric silicone foam (5) after step G) by means of organosilane chemistry.

12. Manufacturing process for a chip (12) containing at least one piece of a macroporous elastomeric silicone foam (5), according to claim 10, comprising the following steps:
I) Fabricating a PDMS housing structure (9) with a reservoir (10) suitable for holding at least one piece of a macroporous elastomeric silicone foam (5), using a suitable casting mold;
J) Fabricating a PDMS cover lid (13) cast from PDMS, into which environmental contact holes (11) are punched;
K) Cutting a molded macroporous elastomeric silicone foam (5) into pieces to fit into the PDMS housing structure (9) of step I) and inserting at least one piece of a macroporous elastomeric silicone foam (5) into the reservoir (10);
L) Plasma surface activation of the PDMS housing structure (9), containing at least one piece of the macroporous elastomeric silicone foam (5) from step K), and of the PDMS cover lid (13).
M) Covering the housing structure (9) containing at least one piece of the macroporous elastomeric silicone foam (5) with the PDMS cover lid (13);
N) Curing the PDMS housing structure including the cover lid and sealing the PDMS housing structure including the cover lid by bonding.

13. Use of the device (1) according to one of claims 1-10 for the enrichment, colonization, cultivation and isolation of microorganisms *in situ* in a wide variety of habitats.

14. Use of the device (1) according to one of claims 1-10 for the enrichment, colonization and isolation of previously uncultivable or unknown microorganisms *in situ* in a wide variety of habitats.

15. Use of the device (1) according to one of claims 1-10 in dry, wet and liquid environments, such as indoor air, soils, moving bed biofilters or wastewater treatment plants.
